# EUROPEAN PATENT SPECIFICATION

(11) **EP 0 411 273 B1**
(45) Date of publication and mention of the grant of the patent: **11.10.1995**
(21) Application number: 90110558.5
(22) Date of filing: 05.06.1990
(51) Int. Cl.: A61B 17/56

(54) **Osteosynthetic assembly with twist fixation plate**
Osteosynthetische Zusammenstellung mit tordierter Befestigungsplatte
Ensemble d'ostéosynthèse avec plaque de fixation torsadée

(30) Priority: 03.08.1989 US 388848
(43) Date of publication of application: 06.02.1991
(73) Proprietor: Synthes AG, Chur, CH-7002 Chur (CH)
(72) Inventor: Frigg, Robert, CH-7270 Davos-Dorf (CH)
(74) Representative: Lusuardi, Werther Giovanni, Dr.

(56) References cited:
- EP-A- 0 192 840
- US-A- 3 025 853
- US-A- 4 103 683
- US-A- 4 733 654

## Description

### Field of the invention

This invention relates to an assembly for use in osteosynthesis according to the preamble of claim 1.

### Background of the invention

The assembly according to the invention is used for the treatment of fractures of the femur which extend into the neck of the bone and which are generally more difficult to treat than fractures restricted to the shaft of the femur.

Various osteosynthetic assemblies have been used in the treatment of these difficult fractures.

In one class of devices an intramedullary nail having transverse holes at or near its head is inserted into the femur and then one or more screws are inserted angularly across the head of the intramedullary nail. Such a nail is shown, for example in EP-A 257,118. A similar device is disclosed in US-Patent No. 4,628,920 MATHYS showing an intramedullary nail with a longitudinal through-slot for receiving transverse wires or pins with the purpose of fixing the head of the intramedullary nail, i.e. in order to give rotational stability to the intramedullary nail inserted in the femur.
All such prior art devices of this class have one deficiency or another. Some require extremely large or heavy screws; or where small transverse screws are employed the device does not provide adequate support.

Another known design is disclosed in US-Patent No. 3,025,853 MASON teaching the use of a twisted blade attached to a retaining plate for further attachment to the bone without implantation of an intramedullary nail. The retaining plate of this device is therefore not supported by or fixed to another osteosynthetic device, such as an intramedullary nail, and depends wholly on its attachment to the bone. This produces an unfavourably long lever arm (formed by the helically twisted blade) acting on the bone.

Another known design is disclosed in US-Patent No. 4,103,683 NEUFELD which teaches the combined use of a femoral nail with a slot for receiving a fixation device, which includes a helically twisted blade. The fixation device is firmly seated in the slot by use of a set screw at a right angle with respect to the nail axis allowing no relative movement between the blade and the nail and no anatomically adequate positioning of the fixation device. Furthermore this design produces an unfavourably long lever arm (formed by the helically twisted blade) acting on the femoral nail.

### Summary of the invention

The invention as claimed is intended to remedy these drawbacks. It solves the problem of how to design an osteosynthetic assembly for reduction and stabilization of this type of difficult femur fractures which extend into the neck of the femur which is strong, light and relatively simple to implant.

In a principle aspect it comprises an osteosynthetic assembly for fixation of fractures of the femur which extend into the neck of the femur comprising the features of claim 1.

The fixation plate used in the assembly according to the invention includes an outer retaining plate which can be attached, as by screws, to the lateral shank of the femur and a blade extending from the retaining plate and designed to extend into the neck of the femur. The blade is twisted helically about 90°,preferably in a continuous way and is adapted to movably pass trough a longitudinal slot in an intramedullary nail which has been driven into the shank of the femur. The longitudinal axis of the blade forms preferably an angle a in the range of 90 - 150° with the plane of the retaining plate and preferably carries a cannula for receiving a guide wire, such as a Kirschner wire.
The retaining plate has one or more screw holes to accommodate screws for fastening the retaining plate to the intramedullary nail. The portion of the blade nearest to the retaining plate is preferably orthogonal to said retaining plate.

When said blade is positioned in the slot of the intramedullary nail the portion of the blade in the slot has its major dimension, W, preferably parallel to the longitudinal axis of the intramedullary nail, and the portion of the blade most remote from the retaining plate has its major dimension, W, preferably transverse to the longitudinal axis of the intramedullary nail.
The fixation device is movably supported by the bottom wall of the slot of the intramedullary nail allowing the angular and/or longitudinal positioning of the helically twisted blade by the action of retaining screws inserted through the hole of the retaining plate for retaining firmly the fixation device to the intramedullary nail.

The main advantages offered by the invention are the following:
a) The assembly according to the invention comprises a blade which is not only firmly attached to the bone but which is additionally secured to the intramedullary nail. The resulting structure is both strong and light. By means of this construction the blade of the assembly according to the invention is able to transmit heavy loads both to the bone and to the intramedullary nail. Nevertheless, due to this stable structure, the blade can be designed in a very flat shape necessitating the removal of only a small volume of bone material upon insertion.
b) In the invention the fixation device is actually fixed to the femur and is only movably supported by the bottom wall of the slot of the femoral nail allowing a precise angular and longitudinal positioning of the helically twisted blade by means of the retaining screws inserted through the screw holes of the retaining plate.
c) The force distribution upon the several structural elements (blade/slot of nail/retaining plate/femoral shaft/retaining screw/femoral nail) in this complex osteosynthetic assembly is far superior to the prior art devices.

The assembly according to the invention is useful for osteosynthetic treatment of intertrochanteric fractures and fractures of the neck of the femur. It may also be used to treat brittle bone, such as those caused by osteoporosis. Since smaller, lighter intramedullary nails may be used with this assembly, there is less damage to the rest of the bone. It is easier to remove than similar prior art assemblies. Furthermore, when this assembly is used, it is often unnecessary to ream out as much bone as is the case with the prior art devices. The same assembly may be used for treatment of fractures of either the left or right femur.

The various features of novelty which characterize the invention are pointed out with particularity in the claims annexed to and forming part of this disclosure. For the better understanding of the invention, its operating advantages and specific objects attained by its use, reference should be had to the accompanying drawings and descriptive matter in which are illustrated and described preferred embodiments of the invention.

### Description of the drawings

Fig. 1 is a schematic view showing an osteosynthetic assembly according to the invention, including a fixation plate and an intramedullary nail implanted in a femur;
Fig. 2 is a more detailed, partially fragmentary, schematic view showing the fixation plate of Fig. 1 inserted in an intramedullary nail;
Fig. 3 is a view in side elevation of the fixation plate of Fig. 1;
Fig. 4 is a view in side elevation of a modified fixation plate used in the invention;
Fig. 4A is a cross sectional view, showing the section A-A of Fig. 4;
Fig. 5 is a schematic view showing insertion of an intramedullary nail into a femur in an osteosynthetic procedure using the assembly according to the invention;
Fig. 6 is a schematic view showing insertion of a guide wire such as a Kirschner wire through the intramedullary nail of Fig. 5;
Fig. 7 is a schematic view showing use of a chisel in an osteosynthetic procedure to cut a hole in the lateral side of the femur at the correct angle for implantation of an assembly according to the invention; and
Fig. 8 is a schematic view showing insertion of the fixation plate of an assembly according to the invention into the fractured femur.

### Detailed description

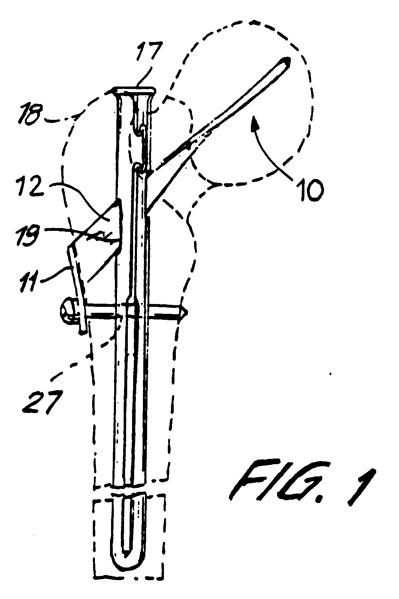

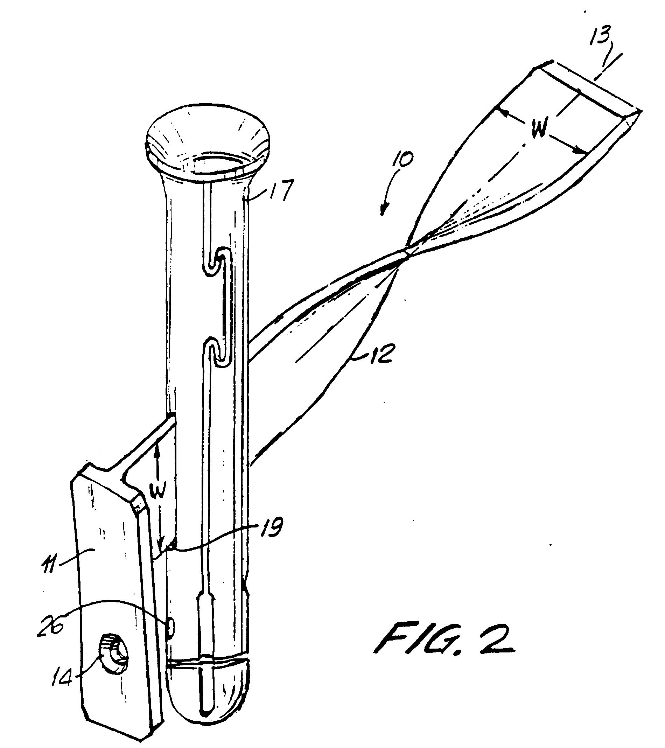

The osteosynthetic assembly according to the invention is shown in Figs. 1 and 2. As shown in Figure 1, an intramedullary nail 17 is positioned in the medulla of a femur 18. The intramedullary nail 17 may be of any suitable design and material, although it is preferred to use an intramedullary nail 17 of the type shown and described in the US-Patent No. 4,628,920. The intramedullary nail 17 is provided with a slot 19 to accommodate the blade 12 of a fixation plate 10 and one or more through holes 26 to accommodate one or more retaining screws 27 inserted through screw hole 14 in the retaining plate 11. Additional screw holes (not shown) may be provided to permit transverse retaining screws or bolts (not shown) to be inserted at the distal end of the intramedullary nail 17.

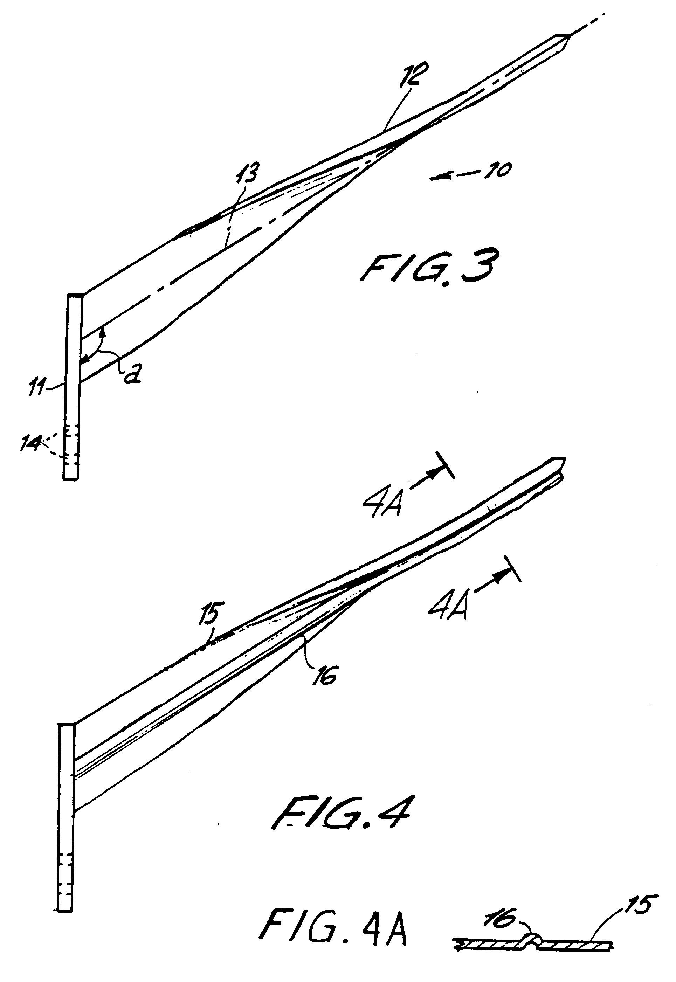

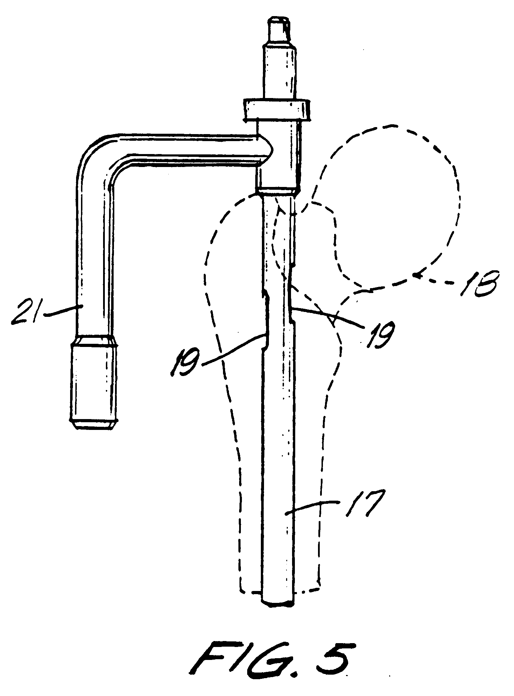

Referring now to Fig. 3, the fixation plate 10 as used in the assembly according to the invention comprises a retaining plate 11 which is adapted to lie along the lateral shaft of the femur 18 and a blade 12 which extends transversely to the plane of retaining plate 11. The axis 13 of the blade 12 forms an angle a with the plane of retaining plate 11 which may vary according to the individual patient, but which will in general be between about 90° and about 150°.
As shown in Fig. 3, the blade 12 is helically twisted around its axis 13, the total angular displacement being about 90°.
The retaining plate 11 may be furnished with one or more screw holes 14 for fixing the retaining plate 11 to the lateral shaft of the femur 18.
A modified version of the fixation plate 10 is shown in Figs. 4 and 4A. Referring to these figures, the blade 15 of the fixation plate 10 may be provided with a cannula 16 for receiving a Kirschner guide wire or the like to facilitate insertion of the blade 15 into the bone, in the manner described below.

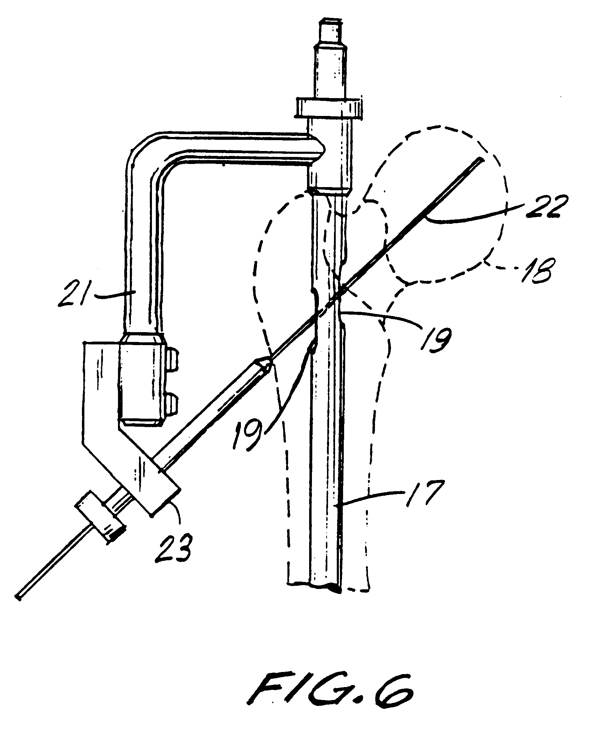

The method of using an osteosynthetic assembly according to the invention is illustrated in Figs. 5 to 8.

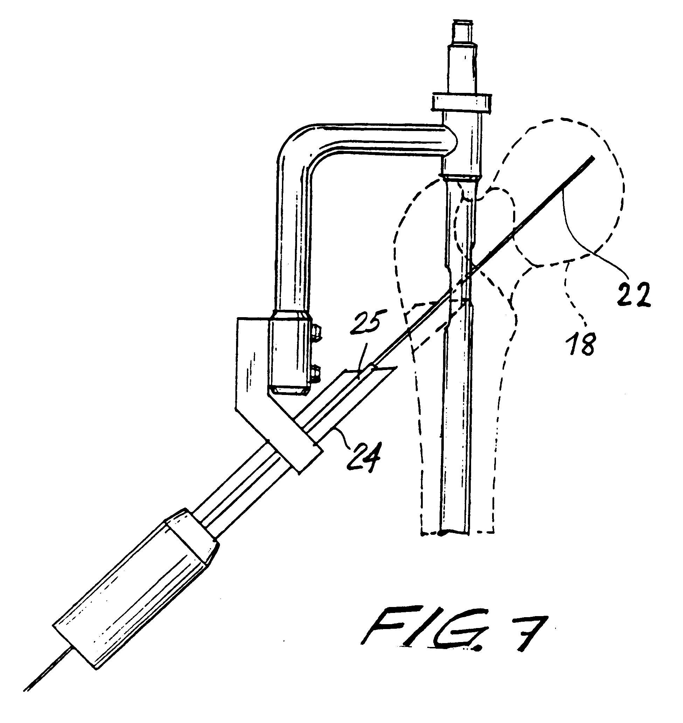

In Fig. 5, an intramedullary nail 17 having the structure shown in Figs. 1 and 2 has been inserted into the lateral shaft of a femur 18, in conventional manner. Attached to the intramedullary nail 17 is an insertion handle 21, a standard fixture used to insert intramedullary nails. Following emplacement of the intramedullary nail 17, as shown in Fig. 6, a Kirschner guide wire 22 may be inserted through the area of the fracture in the neck of the femur 18, passing through slot 19 in the intramedullary nail 17. The Kirschner guide wire 22 may be installed in a conventional manner using an alignment device 23, attached to the insertion handle 21 to ensure that it is placed at the proper angle. The Kirschner guide wire 22 extends from the exterior of the femur 18 through the bone across the fracture or fractures.

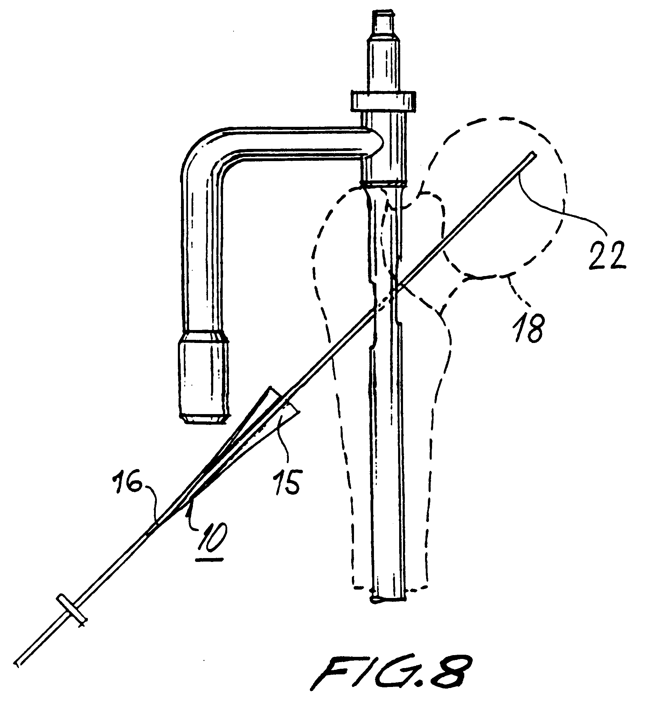

As shown in Figure 7 the Kirschner guide wire 22 may then be used to guide the insertion of a chisel 24 into the bone 18 at the correct angle to cut a hole in the lateral side of the femur 18. The chisel 24 may be cannulated, as at 25, to ensure alignment with the Kirschner guide wire 22.

Figure 8 shows the fixation plate 10 used in the assembly according to the invention about to be inserted into the fractured femur 18. If the blade 15 is cannulated, as shown in Figs. 4 and 4A, the cannula 16 of the fixation plate 10 is slid over the Kirschner guide wire 22 to ensure insertion of the fixation plate 10 at the correct angle. It is not necessary, however, to have such a cannula 16 on the blade 15 of the fixation plate 10.

Referring back to Fig. 1, the fixation plate 10 has been inserted and the outer retaining plate 11 lies against the exterior of the lateral shaft of the femur 18. A screw 27 is inserted through the screw hole 14 in the retaining plate 11, into the bone and through screw hole 26 in the intramedullary nail 17.
Referring to Fig. 2, when the blade 12 is inserted in the intramedullary nail 17 through its slot 19 its major dimension, W, transverse to its axis 13, of the portion of the blade 12 in and near the slot 19 is parallel to the axis of the femur 18 into which the intramedullary nail 17 is inserted. Thus the major bending stress, which will occur at the end of the blade 12 nearest the retaining plate 11, will be borne by the major dimension of the blade 12. On the other hand, at the outer end of the blade 12, its major dimension, W, will be transverse to the axis of the intramedullary nail 17 and to the axis of the femur 18 in which it is inserted. Thus the blade 12 will give maximum support where it is actually needed.
The blade 12 of the fixation plate 10 has a large surface area, yet the fixation plate 10 of the assembly according to the invention is less bulky than prior art devices. Consequently it is able to support even soft bone structure.

## Claims

1. An assembly for use in osteosynthesis, said assembly comprising an intramedullary nail (17) for insertion into the medulla of a femur (18) and a fixation device (10) with a helically twisted blade (12) for insertion into the head of the femur (18), and said nail (17) having a through hole (19) to receive and support the blade (12) of said fixation device (10),
**characterized in that**
A) said fixation device (10) has a retaining plate (11) for positioning along the outside surface of the shank of the femur (18);
B) said helically twisted blade (12) extends from said plate (11);
C) said through hole (19) permits movement of said blade (12) relative to said nail (17), said nail (17) and the retaining plate (11) of said fixation device (10) having cooperative apertures (14,26) for receiving retaining means (27) extending through the aperture (14) of said plate (11), through the bone (18) and into the aperture (26) of said nail (17); and
D) said fixation device (10) is movably supported by the bottom wall of said through hole (19) of said intramedullary nail (17) allowing the angular and/or longitudinal positioning of said helically twisted blade (12) by the action of said retaining means (27) for retaining firmly said fixation device (10) to said intramedullary nail (17).

2. An assembly according to claim 1, characterized in that the blade (12) is helically twisted around its axis (13), preferably in a continuous way, the total angular displacement being about 90°.

3. An assembly according to claim 1 or 2, characterized in that the blade (12) has a longitudinal axis (13) which forms an angle a in the range of 90 - 150° with the plane of said retaining plate (11).

4. An assembly according to one of the claims 1 to 3, characterized in that the blade (15) has a cannula (16) for receiving a guide wire (22).

5. An assembly according to one of the claims 1 to 4, characterized in that the portion of the blade (12;15) nearest to the retaining plate (11) is orthogonal to said retaining plate (11).

6. An assembly according to one of the claims 1 to 5, characterized in that when said blade (12;15) is positioned in the through hole (19) of said intramedullary nail (17) the portion of the blade (12,15) in the through hole (19) has its major dimension generally parallel to the longitudinal axis of said intramedullary nail (17), and the portion of the blade (12,15) most remote from said retaining plate (11) has its major dimension generally transverse to the longitudinal axis of said intramedullary nail (17).

7. An assembly according to one of the claims 1 to 6, characterized in that said means (27) are in form of retaining screws (27) inserted through the said cooperative apertures (14) of said retaining plate (11).

## Patentansprüche

1. Eine Baugruppe zur Verwendung bei der Osteosynthese, welche Baugruppe einen Marknagel (17) für das Einfügen in die Medulla eines Femurs (18) und eine Fixiervorrichtung (10) mit einem schraubenartig verdrillten Blatt (12) für das Einfügen in den Kopf des Femurs (18) umfaßt, wobei der Nagel (17) ein Durchgangsloch (19) zur Aufnahme und Abstützung des Blattes (12) der Fixiervorrichtung (10) aufweist,
**dadurch gekennzeichnet**, daß
A) die Fixiervorrichtung (10) eine Halteplatte (11) für Positionierung längs der Außenoberfläche des Schaftes des Femurs (18) besitzt;
B) das schraubenartig verdrillte Blatt (12) sich von der Platte (11) wegerstreckt;
C) das Durchgangsloch (19) die Bewegung des Blattes (12) relativ zu dem Nagel (17) ermöglicht, wobei der Nagel (17) und die Halteplatte (11) der Fixiervorrichtung (10) zusammenwirkende Öffnungen (14,26) aufweisen für die Aufnahme von Haltemitteln (27), die sich durch die Öffnung (14) der Platte (11) erstrecken durch den Knochen (18) und in die Öffnung (26) des Nagels (17); und
D) die Fixiervorrichtung (10) beweglich von der Bodenwandung des Durchgangsloches (19) des Marknagels (17) abgestützt ist, was eine Winkel- und/oder Längspositionierung des schraubenartig verdrillten Blattes (12) durch die Wirkung der Haltemittel (27) für das sichere Halten der Fixiervorrichtung (10) an dem Marknagel (17) ermöglicht.

2. Eine Baugruppe nach Anspruch 1, dadurch gekennzeichnet, daß das Blatt (12) schraubenartig um seine Achse (13) verdrillt ist, vorzugsweise kontinuierlich, wobei die Gesamtwinkelversetzung um etwa 90° liegt.

3. Eine Baugruppe nach Anspruch 1 oder 2, dadurch gekennzeichnet, daß das Blatt (12) eine Längsachse (13) aufweist, die einen Winkel a im Bereich von 90 - 150° mit der Ebene der Halteplatte (11) bildet.

4. Eine Baugruppe nach einem der Ansprüche 1 bis 3, dadurch gekennzeichnet, daß das Blatt (15) eine Rille (16) für die Aufnahme eines Führungsdrahtes (22) aufweist.

5. Eine Baugruppe nach einem der Ansprüche 1 bis 4, dadurch gekennzeichnet, daß der Abschnitt des Blattes (12,15) nächst der Halteplatte (11) orthogonal zu der Halteplatte (11) ist.

6. Eine Baugruppe nach einem der Ansprüche 1 bis 5, dadurch gekennzeichnet, daß dann, wenn das Blatt (12,15) in dem Durchgangsloch (19) des Marknagels (17) positioniert ist, der Abschnitt des Blattes (12;15) in dem Durchgangsloch (19) mit seiner Hauptabmessung generell parallel zu der Längsachse des Marknagels (17) steht und der Abschnitt des Blattes (12,15), der von der Halteplatte (11) am entferntesten ist, seine Hauptabmessung generell quer zur Längsachse des Marknagels (17) besitzt.

7. Eine Baugruppe nach einem der Ansprüche 1 bis 6, dadurch gekennzeichnet, daß die Mittel (27) die Form von Halteschrauben (27) besitzen, eingefügt durch die zusammenwirkenden Öffnungen (14) der Halteplatte (11).

## Revendications

1. Assemblage destiné à être utilisé dans l'ostéosynthèse, ledit assemblage comprenant une tige intramédullaire (17) destinée à être insérée dans la moelle d'un fémur (18) et un organe de fixation (10) muni d'une lame torsadée hélicoïdalement (12) destiné à être inséré dans la tête du fémur (18), et ladite tige (17) comprenant un orifice traversant (19) apte à recevoir et à supporter la lame (12) dudit organe de fixation (10),
caractérisé en ce que
A) ledit organe de fixation (10) comporte une plaque de retenue (11) destinée au positionnement le long de la surface extérieure du corps du fémur (18);
B) ladite lame torsadée hélicoïdalement (12) s'étend à partir de ladite plaque (11);
C) ledit orifice traversant (19) rend possible le déplacement de ladite lame (12) par rapport à ladite tige (17), ladite tige (17) et la plaque de retenue (11) dudit organe de fixation (10) comprenant des ouvertures associées (14, 26) destinées à recevoir des moyens de retenue (27) s'étendant à travers l'ouverture (14) de ladite plaque (11), à travers l'os (18) et dans l'ouverture (26) de ladite tige (17), et
D) ledit organe de fixation (10) est supporté de façon mobile par la paroi inférieure dudit orifice traversant (19) de ladite tige intramédullaire (17), de manière à permettre le positionnement angulaire et/ou longitudinal de ladite lame torsadée hélicoïdalement (12) sous l'effet desdits moyens de retenue (27) pour retenir fermement ledit organe de fixation (10) à ladite tige intramédullaire (17).

2. Assemblage selon la revendication 1, caractérisé en ce que la lame (12) est torsadée hélicoïdalement autour de son axe (13), de préférence d'une manière continue, le déplacement angulaire total étant d'environ 90°.

3. Assemblage selon la revendication 1 ou 2, caractérisé en ce que la lame (12) a un axe longitudinal (13) qui forme un angle a dans la plage de 90 - 150° par rapport au plan de ladite plaque de retenue (11).

4. Assemblage selon l'une des revendications 1 à 3, caractérisé en ce que la lame (15) comporte une canule (16) destinée à recevoir un fil de guidage (22).

5. Assemblage selon l'une des revendications 1 à 4, caractérisé en ce que la partie de la lame (12, 15) la plus proche de la plaque de retenue (11) est orthogonale à ladite plaque de retenue (11).

6. Assemblage selon l'une des revendications 1 à 5, caractérisé en ce que, lorsque la partie de la lame (12, 15) est positionnée dans l'orifice traversant (19) de ladite tige intramédullaire (17), la partie de la lame (12,15) dans l'orifice traversant (19) a sa plus grande dimension généralement parallèle à l'axe longitudinal de ladite tige intramédullaire (17), et la partie de la lame (12,15) la plus éloignée de ladite plaque de retenue (11) a sa plus grande dimension généralement transversale par rapport à l'axe longitudinal de ladite tige intramédullaire (17).

7. Assemblage selon l'une des revendications 1 à 6, caractérisé en ce que lesdits moyens (27) se présentent sous la forme de vis de retenue (17) insérées à travers lesdites ouvertures associées (14) de ladite plaque de retenue (11).
